# EUROPEAN PATENT APPLICATION

(11) **EP 4 591 868 A1**
(43) Date of publication of application: **30.07.2025**
(21) Application number: 23866823.0
(22) Date of filing: 08.03.2023
(51) Int. Cl.: A61K 31/708, A61P 35/00, A61P 35/04, A61P 35/02

(54) **MEDICAMENT FOR PREVENTING AND TREATING TUMOR AND USE THEREOF**

(30) Priority: 23.09.2022 CN 202211163136
(71) Applicant: Beijing Hospital, Beijing 100730 (CN)
(72) Inventor: CAI, Jianping, Beijing 100730 (CN); LI, Jin, Beijing 100730 (CN); WANG, Zihui, Beijing 100730 (CN)
(74) Representative: Gulde & Partner
(86) International application number: PCT/CN2023/080291
(87) International publication number: WO 2024/060520

(57) **Abstract**

The present invention discloses the application of 06-Methyl-2-deoxyguanosine-5-triphosphate (06-Methyl-dGTP) in the preparation of drugs for the prevention, treatment and/or control of tumours. 06-Methyl-dGTP significantly inhibits tumour proliferation and promotes apoptosis, providing a novel drug candidate for tumour prevention and treatment. Animal experiments confirmed its inhibitory effect on tumours, showing strong industrial applicability as well as significant commercial and social value.

## Description

### Technical Field

The present invention relates to a medicament for the prevention and treatment of tumours, in particular the use of 06-methyl-2-deoxyguanosine 5-triphosphate (06-methyl-dGTP) in the preparation of medicaments for the treatment, prevention and/or control of tumours, and medicaments for the prevention and treatment of tumours containing 06-methyl-dGTP, belonging to the pharmaceutical field.

### Background

Currently, chemotherapy remains the main means of cancer treatment. Among the many chemotherapy drugs, alkylating agents are the earliest used and currently the most widely used anticancer drugs. The current anti-tumor mechanism of alkylating agents is believed to be as follows: alkylating agents spontaneously decompose or undergo enzymatic hydrolysis to generate active electrophilic reagents that attack macromolecules such as DNA, producing a large number of alkylated DNA adduct products. Compared with other products, 06-methylguanine (06meG), which accounts for 8% of the modification amount, is sufficient to cause cell death and exert an anti-tumor cytotoxic effect.

Although current research on alkylating agents has focused on damage caused by 06-MedG on the DNA strand and subsequent cytotoxicity, it is worth noting that free nucleotides are approximately 190-13, 000-fold more sensitive to alkylation damage than nucleotides present on the DNA strand. Heleday' steam microinjected 06-methyl-dGTP into zebrafish embryos, which showed a marked increase in the amount of 06-methyl-dG on the DNA strand and toxicity in the presence of MTH1 inhibitors and/or MGMT inhibitors. The amount of 06-methyl-dG on the DNA strand was significantly increased, which could be toxic to embryos in the presence of MTH1 inhibitor and/or MGMT inhibitor. However, the effect of 06-methyl-dGTP on tumor cells has not been reported. However, the effect of 06-methyl-dGTP on tumor cells has not been reported.

Cancer has become the second leading cause of death worldwide. Although various new treatment methods have emerged, such as molecular targeted therapy and immunotherapy, chemotherapy is still the main method of cancer treatment due to limitations of specific tumor types and treatment costs. Among the many chemotherapy drugs, alkylating agents are the earliest used and currently the most widely used anti-cancer drugs. After alkylating agents intervene in cells or organisms, a large amount of alkylated DNA addition products can be produced. Compared with other products, 06-methylguanine (06meG), which accounts for 8% of the modification amount, is sufficient to cause cell death. 06MeG on the DNA chain can pair with T to induce a G:C to A:T mutation. When DNA mismatch repair (MMR) removes 06MeG, an ineffective mismatch repair cycle will occur, resulting in single-strand and double-strand breaks in the DNA. If further repair is not possible, the apoptosis pathway will be activated, leading to cell death. The body has a variety of alkylation DNA damage repair mechanisms, among which methylguanine methyltransferase (MGMT) can transfer the methyl group in DNA from the 6th position of guanine to the cysteine residue in its active site, and then be degraded itself. Therefore, the expression level of MGMT in cells or tissues is closely related to the clinical therapeutic effect of alkylating agents. If the body's MGMT expression level is high, it will produce a certain degree of resistance to the treatment of alkylating agents. Therefore, it is urgent to explore the different mechanisms of action and resistance mechanisms of alkylating agents and to explore new chemotherapy drugs.

Based on our previous studies, although current research on alkylating agents focuses on the damage caused by 06-MedG on the DNA chain and subsequent cytotoxicity, it is worth noting that free nucleotides are approximately 190 to 13,000 times more sensitive to alkylation damage than nucleotides present in the DNA chain. Therefore, we believe that alkylating agents may act by producing 06-Methyl-dGTP.

There are fewer studies of 06-Methyl-dGTP interventions in cells and animals. Only Heleday's team microinjected 06-Methyl-dGTP into zebrafish embryos and found that the content of 06-Methyl-dG on the DNA strand was significantly increased, and it could be toxic to embryos in the presence of MTH1 inhibitor and/or MGMT inhibitor. However, the effects of 06-Methyl-dGTP on tumor cells and experimental animals have not been reported. Therefore, we prepared 06-Methyl-dGTP drug and intervened it in various tumor cells as well as in tumor-bearing nude mice and mice. We found that 06-Methyl-dGTP significantly reduced tumor cell viability, inhibited cell proliferation and tumor growth in tumor-bearing nude mice and mice, and promoted apoptosis, and the effect was not affected by the expression level of MGMT. Therefore, 06-Methyl-dGTP is superior to alkylating agents and has strong medicinal and social values.

### Contents of the Invention

The technical problem to be solved by the present invention is to provide a drug for preventing and treating tumors and its use.

To achieve the above objectives, the present invention adopts the following technical solutions.

06-Methyl-2-deoxyguanosine-5' -triphosphate (06-Methyl-dGTP) for use in the treatment, prevention, and/or control of tumors.

The treatment, prevention, and/or control of tumours comprises one or more of inhibiting the proliferation of tumour cells, preventing the development of tumours, or promoting the death of tumour cells.

Preferably, the tumors are solid tumors or hematological tumors.

Preferably, the tumor is selected from the group consisting of nervous system tumors, head and neck cancer, nasopharyngeal cancer, oral cavity cancer, thyroid cancer, breast cancer, cervical cancer, endometrial cancer, ovarian cancer, lung cancer, esophageal cancer, gastric cancer, colorectal cancer, liver cancer, pancreatic cancer, bladder cancer, renal cancer, testicular cancer, prostate cancer, bone tumors, hematological system tumors, lymphoma, advanced malignant tumors, unresectable malignant tumors, metastatic malignant tumors, and combinations thereof.

A therapeutic, prophylactic and/or tumor-controlling drug comprising 06-Methyl-2-deoxyguanosine-5-triphosphate (06-Methyl-dGTP).

The drug also contains an excipient or carrier acceptable to the organism.

The drug is an orally administered formulation, an injectable parenteral gastrointestinal administration formulation, a nasal mucosal administration formulation, a transdermal administration formulation, a rectal administration formulation, or a reservoir formulation.

The oral delivery preparations are tablets, capsules, flat capsules, soft capsules, solutions or suspensions; the injectable parenteral delivery preparations are rapid intravenous preparations or continuous infusion preparations; the nasal mucosal delivery preparations are aerosols, sprays, mists or drops; the transdermal delivery preparations are gels, ointments, sustained-release transdermal delivery preparations, liposomal preparations, transdermal patches or transdermal sprays; rectal delivery preparations are suppositories or retention enemas; the reservoir preparations are subcutaneous or intramuscular implant delivery preparations or intramuscular injection delivery preparations. formulations are suppositories or retention enemas; reservoir formulations are subcutaneous or intramuscular implant delivery formulations or intramuscular injection delivery formulations.

A method of treating, preventing and/or controlling a tumor, the method comprising administering to a mammal a therapeutically effective amount of 06-methyl-2-deoxyguanosine-5-triphosphate (06-Methyl-dGTP).

The effective therapeutic dose is 5 µ g-2000 µ g/kg body weight, preferably 200-1000 µ g/kg body weight.

The tumor includes a nervous system tumor, a head and neck cancer, a nasopharyngeal cancer, an oral cancer, a thyroid cancer, a breast cancer, a cervical cancer, an endometrial cancer, an ovarian cancer, a lung cancer, an esophageal cancer, a gastric cancer, a colorectal cancer, a liver cancer, a pancreatic cancer, a bladder cancer, a renal cancer, a testicular cancer, a prostate cancer, a bone tumor, a tumor of a hematological system, a lymphoma; a late-stage malignant tumor, an unresectable malignant tumor, a metastatic malignant tumor and the like.

The advantages of the invention are as follows.

The present invention unexpectedly found that 06-Methyl-dGTP can significantly inhibit the growth of various transplanted nude mice tumors and inhibit the proliferation of tumor cells, which provides a new drug candidate for the prevention and treatment of tumors. In addition, the chemical synthesis of 06-Methyl-dGTP has been standardized, so it has a strong prospect of clinical application and social value.

### Brief Description of the Drawings

Specific embodiments of the present invention are described in further detail below in conjunction with the accompanying drawings.
Figure 1 shows the changes in cell viability after 06-Methyl-dGTP intervention in cells of different origins
Figure 2 shows the changes in cell viability after 06-Methyl-dGTP intervention in different glioma cells
Figure 3 shows the effect of detecting 06-Methyl-dGTP on Hela cell viability after knockdown of MGMT
Figure 4 shows that 06-Methyl-dGTP blocks cell cycle progression, the
Figure 5 shows the increase in apoptosis after 06-Methyl-dGTP intervention in tumor cells, the
Figure 6A shows that 06-Methyl-dGTP inhibits tumor growth in CT26 transplanted tumor nude mice
Figure 6B shows that 06-Methyl-dGTP and temozolomide inhibit tumor growth in U251 xenograft tumor nude mice
Figure 6C shows that 06-Methyl-dGTP inhibits tumor growth in Ln229 xenograft tumor nude mice
Figure 7 shows the changes in cell viability after 24h of intervention of U251 cells with different concentrations of 8-oxodGTP and 06-Methyl-dGTP

### Examples

### Example 1: A drug containing 06-Methyl-dGTP

### I. Experimental materials

1. Active ingredient: 06-methyl-2-deoxyguanosine-5-triphosphate (06-Methyl-dGTP), commissioned by Tianjin WuXi AppTec New Drug Development Co. , Ltd. to synthesize.
2. Drug vector (carrier) : Entranster^{™} -in Vivo Reagent, Cat. No. 18668-11, Size:1ml, from Engreen Biosystem Co, Ltd. (Engreen Biosystem), purchased from Beijing Engreen Biosystem Co, Ltd; Thermo Fisher Scientific' s Lipofectamine^{™} 3000 Transfection Reagent, Cat. No. L3000001, Size: 1. 5mL, purchased from Beijing Huashengjian Biotechnology Co.

### II. Methods of preparation

The drug of the invention was obtained by configuring the active ingredient and the drug carrier into a transfection complex according to the manuals of the two drug carriers, in which the concentration of the active ingredient, 06-methyl-2-deoxyguanosine-5-triphosphate (06-Methyl-dGTP), was 0.5 µg/µl.

### Example 2: Tumor prevention and treatment cell experiment

### I. Experimental materials

1. Experimental reagents:
   (1) 06-Methyl-dGTP injection: the drug obtained in Example 1.
   (2) Blank vector reagent: Lipofectamine^{™} 3000 transfection reagent from Thermo Fisher Scientific, prepared according to the method of Example 1, and replace 06-Methyl-2-deoxyguanosine-5-triphosphate (06-Methyl-dGTP) with Opti-M, which is the vector solution without active ingredient.
   (3) Cell Viability Assay Reagent: CellTiter-Glo^{®} Luminescent Cell Viability Assay Reagent, Cat. G7571, Size: 10×10 ml, purchased from Beijing Zhaosheng Leibao Trading Co.
   (4) Apoptosis detection reagent: Annexin V-FITC Apoptosis Detection Kit from Biyuntian Biotechnology, Cat: C1062M, purchased from Beijing Benovi Biotechnology Co.
   (5) Cell cycle detection reagent: Cell cycle and apoptosis detection kit from Biyuntian Biotechnology, Cat: C1052, purchased from Beijing Benovi Biotechnology Co.
2. Experimental cells:
   A549, HCT116 were purchased from ATCC; MCF7, SW480, HepG2, U251 were purchased from Peking Union Medical College Cell Resource Centre, LN229 and U87MG were purchased from Beijing Xinshengke Technology Co.

### II. Experimental methodology

### 1. Detecting the effect of 06-Methyl-dGTP on tumour cell viability

Take 5×10³ cells and spread them in 96-well plate, after 24 hours of adherent growth, use Lipofectamine^{™} 3000 to transfect different concentrations of 06-Methyl-dGTP into the tumour cells, and continue to culture for 48 hours. The cell culture plate was left at room temperature for 30 minutes, 100 µ 1 of cell culture medium was added with 100 µl of cell viability assay reagent, and the contents were mixed on a fixed-track oscillator for 2 minutes to induce cell lysis, while the plate was then incubated at room temperature for 10 minutes to stabilise the luminescence signal, and then the plate was tested on the machine and the signal values were recorded.

### 2. Flow Cytometry Analysis of the Effect of 06-Methyl-dGTP on Tumor Cell Cycle

1×10⁵ cells were spread in a 24-well plate and grown adherently for 24 h. After that, the cells were transfected with different concentrations of 06-Methyl-dGTP into the tumor cells using Lipofectamine^{™} 3000 and the culture was continued for 48 h. The cells were collected and resuspended by adding about 1 ml of ice-bath pre-cooled PBS and transferred to a 1.5 ml centrifuge tube. The cells were collected, and about 1 ml of ice-bath pre-cooled PBS was added to resuspend the cells and transferred to a 1.5 ml centrifuge tube. The cells were precipitated by centrifugation again, and the supernatant was carefully aspirated. 1 ml of ice-bath pre-cooled 70% ethanol was added, gently blown to mix, and fixed at 4°C for 2 hours, then subjected to propidium iodide staining, and finally detected on the machine using flow cytometry.

### 3. Flow cytometry to detect the effect of 06-Methyl-dGTP on apoptosis in tumor cells

Lay 1 × 10⁵ cells on a 24 well plate, adhere to the wall and grow for 24 hours, then use Lipofectamine^{™} 3000 transfected with different concentrations of 06-Methyl-dGTP into tumor cells, and continued to culture for 48 hours. Collect the cells, use PBS to gently resuscitate the cells and count them, take 50000 to 100000 cells, centrifugate 1000g for 5 minutes, discard the supernatant, add 195 µ l Annexin V-FITC binding solution to gently resuscitate the cells, add 5 µ l Annexin V-FITC, add 10 µ l propidium iodide staining solution, and gently mix. Incubate at room temperature and away from light for 10-20 minutes, and then test immediately.

### III. Results of the experiment

### 1.06-Methyl-dGTP inhibits tumor cell growth

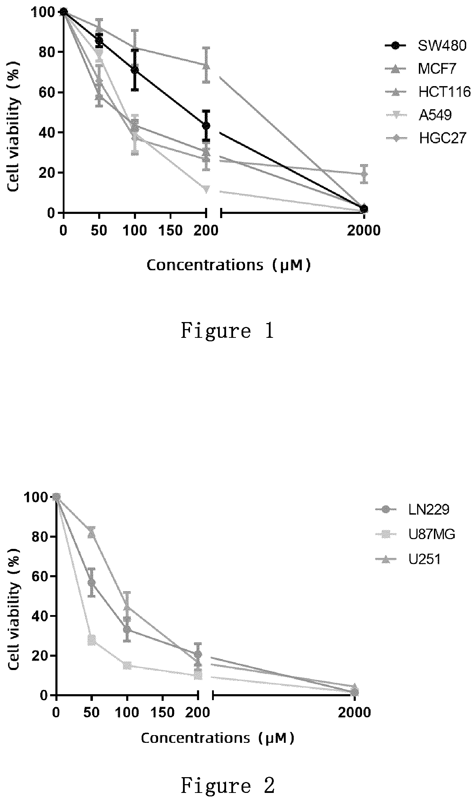

As shown in Figure 1 and Figure 2, after the intervention of different concentrations of 06 Methyl dGTP on different types of tumor cells for 48 hours, the cell viability decreased significantly in a dose-dependent manner, and the most obvious effect could reach nearly 90% inhibition rate after the intervention of 200µ M06-Methyl-dGTP.

### 2. The effect of 06-Methyl-dGTP on tumor cells is independent of MGMT expression

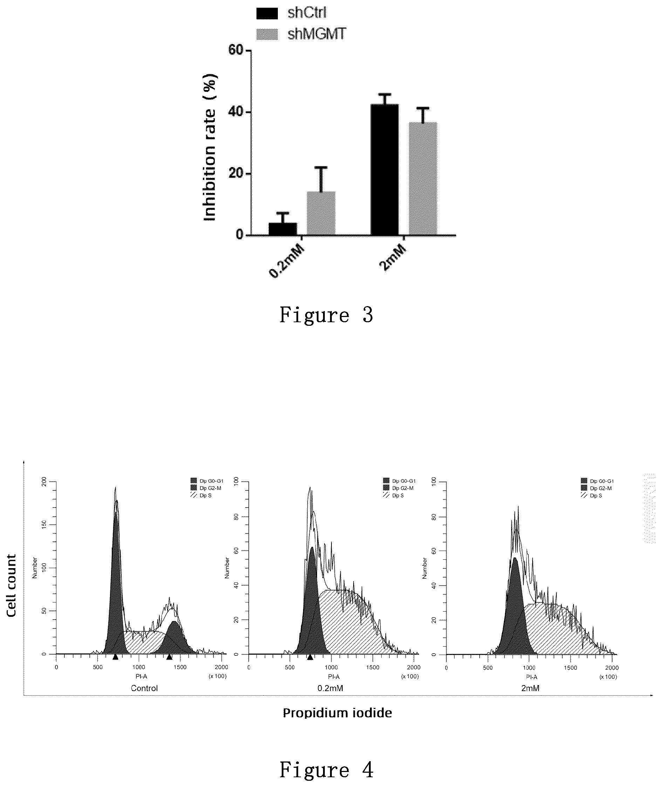

As shown in Figure 3, the level of MGMT expression hardly changed the effect of 06-Methyl-dGTP on tumor cell viability.

### 3. 06-Methyl-dGTP blocks tumor cell cycle progression

As shown in Figure 4, the cell cycle of U251 cells was blocked in S-phase after 48 hours of intervention with different concentrations of 06-Methyl-dGTP, in which the proportion of cells in S-phase was nearly doubled compared with that of the control group after the intervention with 0.2 mM 06-Methyl-dGTP.

### 4. 06-Methyl-dGTP induces apoptosis in tumor cells

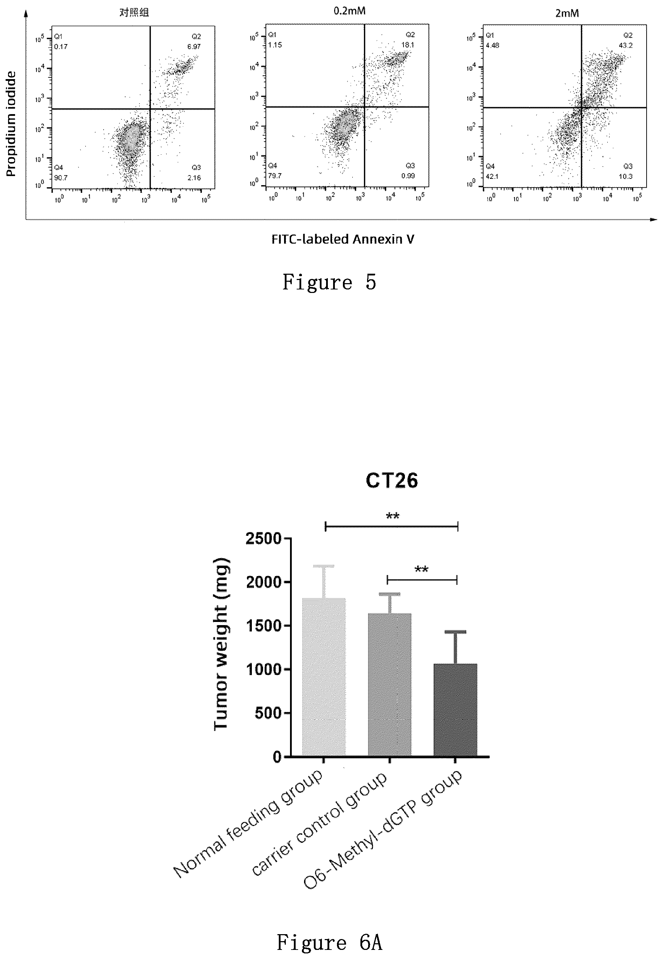

As shown in Figure 5, apoptosis was increased in U251 cells after 48 h of intervention with different concentrations of 06-Methyl-dGTP. 0.2 mM 06-Methyl-dGTP group showed more than 2-fold increase in apoptotic cells compared with the control group, and the proportion of apoptotic cells in the 2 mM 06-Methyl-dGTP group was more than 6-fold of that in the control group, which showed that 06-Methyl-dGTP could induce apoptosis in tumor cells. dGTP induced apoptosis in tumor cells.

### Example 3: Animal experiments for tumor control

### I. Experimental materials

1. Experimental reagents.
   (1)06-Methyl-dGTP Injection: Drug obtained from Example 1
   (2)Blank carrier reagent: EntransterTM -in Vivo reagent from Engreen Biosystem Co, Ltd. was prepared according to the method of Example 1, and 06-Methyl-2-deoxyguanosine-5-triphosphate (06-Methyl-dGTP) was replaced with pure water, which is the carrier that does not contain active ingredient solution.
2. Experimental Animals:
   Male Balb/c nude rats and mice, 17-19g, 6 weeks old, purchased from Beijing Weitong Lihua Experimental Animal Technology Co., Ltd.

All mice involved in the experiment were housed under specific pathogen-free (SPF) animal husbandry standards, with a controlled room temperature of 20-26° C, humidity of 50%-60%, and a 12-hour light-dark cycle. The mice were fed a sterilized standard diet and had free access to food and water.

### 3. Experimental cells.

CT26 cells and LN229 cells were purchased from ATCC, and U251 cells were purchased from Peking Union Medical College Cell Resource Center.

### II. Experimental methods

Tumor inoculation, injection and dissection in nude mice.
1. 1x10⁶ CT26 cells (200 µ 1) were inoculated subcutaneously into the armpit back of mice. After the tumor block grew to a certain size, the tumor block was inoculated subcutaneously into 21 mice. They were randomly divided into normal feeding group and blank carrier control group, In 06-Methyl-dGTP group, mice were administered via tail vein on the second day after the second inoculation. The dosage of 06 Methyl dGTP group was 2.5 mg/kg/time, and the carrier control group was injected with blank carrier reagent of equal volume. Inject at D0, 1, 2, 4, 6, 8, 10 and 12 for 8 times. After the injection, take the tumor block, weigh the tumor, and calculate the inhibition rate (inhibition rate=(1-T experimental group/C control group) × 100%)
2. Inoculate 1x10 ⁷ U251 cells (200 µ 1) under the skin of the armpit back of the nude mice. After the tumor block grows to a certain size, inoculate the tumor block again. After it grows to a certain size, inoculate the tumor block again. After it grows to a certain size, inoculate the tumor block under the flank skin of 18 nude mice. When the tumor size is greater than 3 × 2mm, randomly divide it into a blank carrier control group, 06 Methyl dGTP group and TMZ group began to be administered via tail vein every two days. The dosage of 06 Methyl dGTP and TMZ group was 2. 5 mg/kg/time, and the carrier control group was injected with blank carrier reagent of the same volume. Inject at D0, 3, 6, 9, 12 and 15 for 6 times. After the injection, take the tumor block, weigh it, and calculate the inhibition rate (inhibition rate=(1-T experimental group/C control group) × 100%)
3. 5 × 10 ⁶ Ln229 (200 µ 1) cells were inoculated subcutaneously into the armpit back of nude mice. After the tumor block grew to a certain size, the tumor block was inoculated subcutaneously into 12 nude mice for the second time. When the tumor size was greater than 3 × 2mm, they were randomly divided into a blank carrier control group, 06-Methyl-dGTP group, the nude mice were given tail vein drugs. The dosage of 06 Methyl dGTP group was 2.5 mg/kg/time, and the carrier control group was injected with blank carrier reagent of equal volume. Inject at D0, 2, 4, 6 and 8 for 5 times. After the injection, take the tumor block, weigh the tumor, and calculate the inhibition rate (inhibition rate=(1-T experimental group/C control group) × 100%)

### III. Results of the experiment

### 06-Methyl-dGTP inhibits tumor mass growth in nude mice and mice

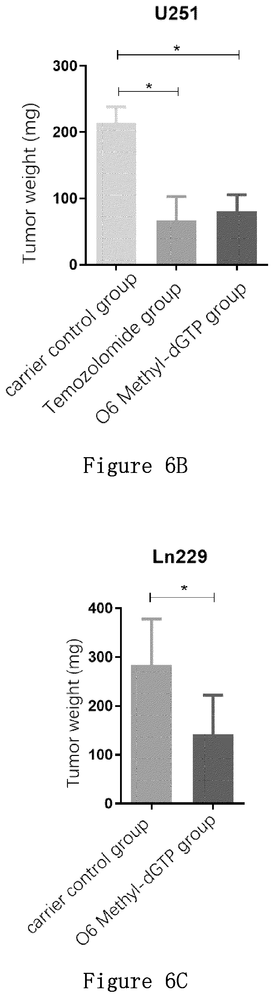

As shown in Tables 1, 2, and 3 with Figures 6A, 6B, and 6C, the inhibition rates of CT26, U251, and Ln229-derived xenograft tumors by 06-Methyl-dGTP were 41. 32%, 68. 77%, and 50. 15%, respectively, as compared with that of the vector control.

**Table 1. tumor weight and inhibition rate in CT26 mice**

| Groups | Tumor weight (mg) | inhibition rate |
|---|---|---|
| Normal feeding group | 1819±367.22 | 0% |
| carrier control group | 1642±220.66 | 9.66% |
| O6-Methyl-dGTP group | 1067±363.08 | 41.32% |

**Table 2. tumor weight and inhibition rate in U251 nude mice**

| Groups | Tumor weight (mg) | inhibition rate |
|---|---|---|
| carrier control group | 214±140.43 | 0% |
| O6-Methyl-dGTP group | 66.83±36.01 | 68.77% |
| TMZ | 80.83±24.90 | 62.22% |

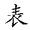 3. Ln229 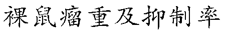

**Table 3. Tumor weight and inhibition rate in Ln229 nude mice**

| Groups | Tumor weight (mg) | inhibition rate |
|---|---|---|
| carrier control group | 284.5±93.61 | 0% |
| O6-Methyl-dGTP group | 141.83±80.05 | 50.15% |

### IV. conclusions of the experiment

The results of cell and animal experiments showed that cell growth was inhibited and apoptosis increased after 06-Methyl-dGTP was administered to intervene in tumors, and tumor growth was slowed down in nude mice and mice, which suggests that 06-Methyl-dGTP has the effect of inhibiting tumor proliferation and promoting apoptosis of tumor cells.

### Example 4:comparative experiment

8-oxodGTP and 06methyl-dGTP are two different small molecules, the main difference is that the base modification sites are different, resulting in different types of mutations, different nucleic acid damage repair enzymes and repair mechanisms, and different effects as tumor suppressor drugs.

### 1. Different base modification sites

8-oxodGTP is generated by oxidation at the C8 position of guanine, while 06methyl-dGTP is generated by methylation at the 06 position of guanine.
(1) The structural formula of 8-oxodGTP is as follows: 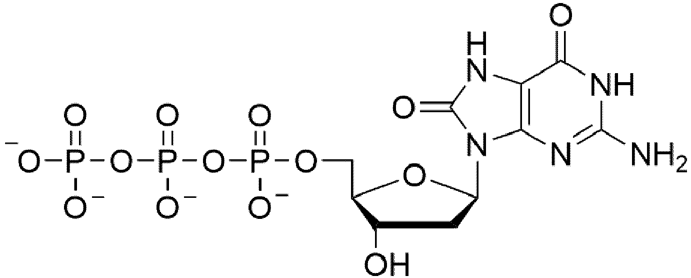
(2) 06methyl-dGTP has the following structural formula.

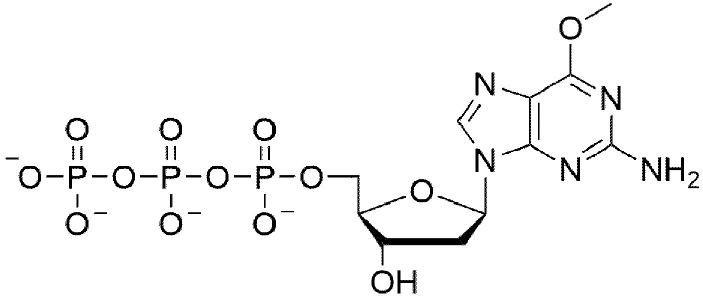

### 2. The types of mutations that result are different

8-oxodGTP can be paired with adenosine dA on the template by incorporation of DNA polymerase λ, and its incorporation into dA is the same as that on the opposite side of dC, resulting in mutations from A: T to C: G and G: C to T: A, while 06methyl-dGTP can be incorporated into the DNA chain and paired with dT, resulting in mutations from G: C to A: T, and the preference of paired dT is 20 times that of paired dC.

### 3. Different nucleic acid damage repair enzymes and repair mechanisms are involved

After 8-oxodGTP was incorporated into the DNA strand, it paired with adenine A and cytosine C. Human 8-hydroxyguanine DNA glycosylase 1 (0GG1) preferentially recognized and excised the 8-oxoG paired with C, initiating base excision repair (BER). Mammalian MutY homolog (MUTYH) removes A mismatched with 8-oxoG, and if C is incorporated into 8-oxoG, OGG1 continues to remove 8-oxoG paired with C, resulting in ineffective base excision repair and strand breakage.

While 06methyl-dGTP can be excised by 06-methylguanine DNA methyltransferase (MGMT) after doping into the DNA strand, if it is not repaired, 06meG:T can be recognized by the mismatch repair system to remove T from the nascent strand, and in the next cycle, if 06meG is paired with T again, it can produce an ineffective mismatch repair cycle, resulting in DNA strand breaks.

### 4. Differential effects

After intervening U251 cells with 0.05, 0.1, 0.2, and 2 mM 8-oxodGTP and 06methyl-dGTP for 24 h, respectively, changes in cell viability were detected using CellTiter-Glo^{®} Luminescent Cell Viability Assay (Promega, G7571) kit.

The experimental methodology was as follows.

5×10³ cells were spread in a 96-well plate and grown adherent for 24 h. After that, Lipofectamine^{™} 3000 was used to transfect different concentrations of 8-oxodGTP and 06-Methyl-dGTP into U251 cells, and the culture was continued for 24 hours. Mix CellTiter-Glo^{®} Luminescent Cell Viability Assay (CTG) Reagent A and B. Remove the 96-well plate and equilibrate at room temperature for 30 minutes, then add 100uL of CTG Reagent to each well, and shake on a shaker for 2 minutes, equilibrate away from light for 10 minutes, and then test on the machine. (CTG reagent is purchased from Promega, No. G7571).

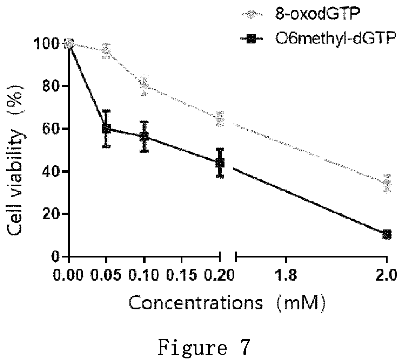

The results are shown in Figure 7. It can be found that the inhibitory effect of 06methyl-dGTP on U251 cells was significantly better than that of 8-oxodGTP after the same concentration intervention for the same time.

The series of detailed descriptions set forth above are merely specifications for feasible embodiments of the present invention, and they are not intended to limit the scope of protection of the present invention, and many other modifications and embodiments may be devised by those skilled in the art, which will fall within the scope and spirit of the principles disclosed in the present application. More specifically, within the scope of the present application disclosure, the accompanying drawings, and the claims, a variety of variations and improvements can be made to the constituent parts and/or layouts of the subject combination layout. In addition to the variations and improvements to the constituent parts and/or layouts, other uses will be apparent to those skilled in the art.

## Claims

1. 06-Methyl-2-deoxyguanosine-5'-triphosphate (06-Methyl-dGTP) for use in the treatment, prevention, and/or control of tumors.

2. 06-Methyl-2-deoxyguanosine-5'-triphosphate (06-Methyl-dGTP) for use according to claim 1, wherein the treatment, prevention, and/or control of tumors comprises one or more of inhibiting the proliferation of tumor cells, preventing the occurrence of tumors, or promoting the death of tumor cells.

3. 06-Methyl-2-deoxyguanosine-5'-triphosphate (06-Methyl-dGTP) for use according to claim 1 or 2, wherein the tumors are solid tumors or hematological tumors.

4. 06-Methyl-2-deoxyguanosine-5'-triphosphate (06-Methyl-dGTP) for use according to claim 3, wherein the tumor is selected from the group consisting of nervous system tumors, head and neck cancer, nasopharyngeal cancer, oral cavity cancer, thyroid cancer, breast cancer, cervical cancer, endometrial cancer, ovarian cancer, lung cancer, esophageal cancer, gastric cancer, colorectal cancer, liver cancer, pancreatic cancer, bladder cancer, renal cancer, testicular cancer, prostate cancer, bone tumors, hematological system tumors, lymphoma, advanced malignant tumors, unresectable malignant tumors, metastatic malignant tumors, and combinations thereof.

5. A drug for the treatment, prevention and/or control of tumors, **characterized in that** it: contains 06-Methyl-2-deoxyguanosine-5-triphosphate (06-Methyl-dGTP).

6. A drug for the treatment, prevention and/or control of tumors according to claim 5, **characterized in that**: said drug further contains an excipient or carrier acceptable to the organism.

7. A drug for the treatment, prevention and/or control of tumors according to claim 5 or 6, **characterized in that**: said drug is an orally administered formulation, an injected parenteral gastrointestinal administration formulation, a nasal mucosal administration formulation, a transdermal administration formulation, a rectal administration formulation, or a reservoir formulation.

8. A drug for the treatment, prevention and/or control of tumors according to claim 7, **characterized in that**: said oral administration preparation is a tablet, capsule, flat capsule, soft capsule, solution or suspension; said parenteral administration preparation for injection is a rapid intravenous preparation or a continuous infusion preparation; said nasal mucosal administration preparation is an aerosol, a spray, an aerosol, a mist, or a liquid drop; said transdermal administration preparation is a gel, ointment, extended-release transdermal delivery formulation, liposomal formulation, transdermal patch or transdermal spray formulation; rectal delivery formulations are suppositories or lagoon enemas; reservoir formulations are subcutaneous or intramuscular implant delivery formulations or intramuscular injection delivery formulations.

9. A method of treating, preventing and/or controlling a tumor, the method comprising administering to a mammal a therapeutically effective amount of 06-methyl-2-deoxyguanosine-5-triphosphate (06-Methyl-dGTP).

10. The method for treating, preventing and/or controlling tumors according to claim 9, which is **characterized in that** the effective therapeutic dose is 5 µ g-2000 µ g/kg body weight, preferably 200-1000 µ g/kg body weight.

11. The method of treating, preventing and/or controlling a tumor according to claim 9 or 10, **characterized in that**: said tumor comprises a tumor of the nervous system, head and neck cancer, nasopharyngeal cancer, oral cancer, thyroid cancer, breast cancer, cervical cancer, endometrial cancer, ovarian cancer, lung cancer, esophageal cancer, gastric cancer, colorectal cancer, hepatocellular carcinoma, pancreatic cancer, bladder cancer, renal carcinoma, testicular carcinoma, prostatic carcinoma, bone tumors, blood system tumors, lymphoma; advanced malignant tumors, unresectable malignant tumors, metastatic malignant tumors and so on.
